# EUROPÄISCHE PATENTSCHRIFT

(11) **EP 4 431 247 B1**
(45) Veröffentlichungstag und Bekanntmachung des Hinweises auf die Patenterteilung: **10.12.2025**
(21) Anmeldenummer: 24157925.9
(22) Anmeldetag: 15.02.2024
(51) Int. Cl.: B25J 1/08, A61L 2/00, B25J 18/04, B25J 19/00, F16K 41/00, A61L 2/16, A61L 2/20

(54) **HUBVORRICHTUNG ZUR VERWENDUNG IN EINEM STERILEN ISOLATIONSBEREICH, ISOLATOR UND PRODUKTIONSANLAGE SOWIE VERFAHREN ZU DEREN BETRIEB**
LIFTING DEVICE FOR USE IN A STERILE INSULATION AREA, ISOLATOR AND PRODUCTION SYSTEM AND METHOD FOR OPERATING SAME
DISPOSITIF DE LEVAGE DESTINÉ À ÊTRE UTILISÉ DANS UNE ZONE D'ISOLATION STÉRILE, ISOLATEUR ET INSTALLATION DE PRODUCTION AINSI QUE SON PROCÉDÉ DE FONCTIONNEMENT

(30) Priorität: 14.03.2023 DE 102023106275
(43) Veröffentlichungstag der Anmeldung: 18.09.2024
(73) Patentinhaber: Syntegon Technology GmbH, 70372 Stuttgart (DE)
(72) Erfinder: Bäuerle, Johannes, 74423 Obersontheim (DE); Ilgenfritz, Markus, 91555 Feuchtwangen (DE); Nagler, Stefan, 73485 Unterschneidheim (DE)
(74) Vertreter: Novagraaf Group

(56) Entgegenhaltungen:
- EP-B1- 1 200 313
- DE-A1- 102021 101 402
- DE-C1- 19 905 831
- US-B2- 11 548 172

## Beschreibung

Bei in der Pharma-, Lebensmittel-, und/oder Medizintechnikindustrie eingesetzten Produktionsanlagen ist es regelmäßig von Bedeutung, dass im Verlauf eines Produktionsprozesses eine sterile Handhabung der entsprechenden Produkte gewährleistet ist. Beispielsweise kann dies ein steriles Abpacken oder Abfüllen von pharmazeutischen oder medizintechnischen Erzeugnissen sein.

Zu diesem Zweck weisen entsprechende Produktionsanlagen typischerweise einen sogenannten Isolator mit einem nach außen geschlossenen Isolationsbereich auf. Der Isolationsbereich bzw. der Isolator bildet dabei eine geschlossene Umgebung mit einer eigenen Atmosphäre, die speziellen Anforderungen an Reinheit und Freiheit von Kontaminationen genügt. In anderen Worten bildet der Isolator einen von der nichtsterilen Umgebung getrennten Bereich aus, in welchem eine sterile Handhabung der Produkte ermöglicht ist. Geschlossen in diesem Sinne bedeutet, dass eine gewisse Trennung zwischen dem Isolationsbereich und seiner Umgebung gegeben ist. Die Trennung kann hermetisch sein, bspw. um hohen Reinheitsanforderungen bzw. Vermeidung von Kontaminationen zu genügen, die Trennung kann aber bspw. auch durch einen Überdruck im Isolationsbereich gegeben sein, der durch Öffnungen in dem Gehäuse, das den Isolationsbereich umgibt, nach außen entweicht. Durch den Überdruck wird ein Eintrag von Verschmutzungen in den Isolationsbereich verhindert bzw. stark reduziert.

Der Isolationsbereich ist zumindest durch ein Trennelement von einem nichtsterilen Bedienbereich getrennt, in welchem sich Bedienpersonal aufhalten kann. Das Trennelement ist dabei typischerweise Teil des Gehäuses, das den Isolationsbereich umgibt. In den meisten Fällen handelt es sich bei dem Trennelement um eine Tischplatte.

In Abhängigkeit des jeweiligen Anwendungsfalls ist es bei gängigen Produktionsanlagen oftmals nötig, die Produkte, Objekte und/oder Vorrichtungen, die im Verlauf des Produktionsprozesses benötigt werden, innerhalb des Isolators zu verlagern. Regelmäßig ist hierbei eine Verlagerung in Vertikalrichtung, also ein Anheben und Absenken bzw. eine Hubbewegung, notwendig. Um dies zu gewährleisten, ist regelmäßig eine sogenannte Hubvorrichtung vorgesehen, die bei bestimmungsgemäßem Gebrauch im Bereich des Trennelements angeordnet ist. Die Hubvorrichtung weist eine Hubstange auf, die - relativ zum Trennelement gesehen - durch das Trennelement bzw. eine entsprechende Öffnung des Trennelements hindurch in Aufwärts- und Abwärtsrichtung verlagerbar ist, also dazu ausgebildet ist, die besagte Hubbewegung durchzuführen. Insofern verfährt die Hubvorrichtung bzw. Hubstange regelmäßig von dem unsterilen Bereich in den sterilen Isolationsbereich hinein.

Durch die Bewegung der Hubstange ergibt sich hierbei die Problematik, dass die Gefahr einer Verschleppung von Kontaminationen in den sterilen Isolationsbereich hinein besteht, sodass eine erhebliche Beeinträchtigung der Sterilität drohen kann.

Um dies zu vermeiden, ist es bekannt, die Hubstange zumindest bereichsweise mit einem an dem Trennelement anordenbaren Faltenbalg zu ummanteln, um den sterilen von dem unsterilen Bereich zu entkoppeln. In anderen Worten stellt der Faltenbalg eine flexible Schutzhülle für die Hubstange dar. Der Faltenbalg bildet hierbei im bestimmungsgemäß montierten Zustand eine flexible Verlängerung des Trennelements aus. Der Faltenbalg kann sich synchron zu der Hubbewegung der Hubstange derart verformen, dass im Wesentlichen ständig eine Entkopplung des sterilen von dem nichtsterilen Bereich gewährleistet ist. Der Faltenbalg macht die Hubbewegung also mit. Insofern ist der Faltenbalg bei bestimmungsgemäßem Gebrauch bzw. vorgesehener Montage der Hubvorrichtung an dem Trennelement montiert.

Bei bekannten Produktionsanlagen ist der Faltenbalg oberhalb des Trennelements angeordnet, also innerhalb des sterilen Isolationsbereichs bzw. Isolators.

Allerdings ist diese Anordnung bei derzeit bekannten Anwendungsfällen aufgrund von gegebenenfalls notwendigen großen Hüben, Platzmangel innerhalb des Isolators und/oder kombinierten Hub-Schwenkbewegungen nicht immer möglich. Es kommt insofern regelmäßig zu einem Platzproblem. Zudem birgt die bekannte Anordnung des Faltenbalgs innerhalb des Isolators die Gefahr, dass bei etwaigen Undichtigkeiten der Hülle des Faltenbalgs kontaminierte Luft in den sterilen Isolationsbereich eindringen kann. Da der Faltenbalg im Verlauf der Hubbewegung der Hubstange langgezogen wird, ergibt sich ein Unterdruck, durch welchen im Fall von Undichtigkeiten kontaminierte Luft von außen in einen zwischen der Hubstange und dem Faltenbalg ausgebildeten Hohlraum angesaugt wird. Wird der Faltenbalg im Anschluss wieder gestaucht, so wird die zuvor angesaugte und nunmehr im Hohlraum befindliche Luft aus diesem heraus gedrückt, wobei sie im Falle von Perforationen der Hülle entsprechend ins Innere des Isolators gepresst wird.

Darüber hinaus ist bei der bekannten Anordnung des Faltenbalgs innerhalb des Isolators dessen Reinigung vergleichsweise aufwendig. EP1200313B1 offenbart diesbezüglich eine Wanddurchführungsvorrichtung zur Übertragung von längsgerichteten Stellbewegungen aus einer unsterilen Umgebung auf der einen Wandseite in eine sterile Umgebung auf der anderen Wandseite, insbesondere in eine Sterilkammer, durch einen eine Wanddurchführungsöffnung durchgreifenden, axial verschieblichen Stößel. Die Wanddurchführungsvorrichtung schirmt den Bereich des Stößels, welcher im Zuge seiner Betätigung seine Lage zwischen der sterilen und der unsterilen Wandseite wechselt, sowohl gegenüber der sterilen, als auch gegenüber der unsterilen Umgebung ab, so daß Keimverschleppungen aus der unsterilen Umgebung in die sterile Umgebung ausgeschlossen sind. DE102021101402A1 offenbart einen Isolator zur Verarbeitung von medizinischen Stoffen sowie ein Verfahren zur Dekontamination eines Isolators.

Die Aufgabe der vorliegenden Erfindung ist es, eine demgegenüber verbesserte Lösung bereitzustellen, bei welcher die vorstehend erörterten Problematiken im Wesentlichen vermieden werden, zumindest jedoch verringert sind. Es soll also eine Hubvorrichtung bereitgestellt werden, die Sterilität des Isolationsbereichs gewährleistet und dabei zuverlässig und wartungsarm ist.

Die Aufgabe wird gelöst durch eine Hubvorrichtung gemäß Anspruch 1, einen Isolator nach Anspruch 2, eine Produktionsanlage nach Anspruch 3 und ein Verfahren nach Anspruch 12.

Die erfindungsgemäße Hubvorrichtung ist zur Verwendung in einem sterilen Isolationsbereich ausgebildet, wobei der sterile Isolationsbereich jedenfalls bereichsweise durch ein Trennelement, dass insbesondere als Tischplatte ausgebildet ist, von einem nichtsterilen Bedienbereich getrennt ist.

Die Hubvorrichtung weist zumindest eine Hubstange mit einem ersten Ende und einem zweiten Ende auf. Das erste Ende ist im montierten Zustand zur Anordnung in dem sterilen Isolationsbereich und das zweite Ende zur Anordnung außerhalb des Isolationsbereichs vorgesehen. Unter dem montierten Zustand ist im Zusammenhang mit der vorliegenden Erfindung der verbaute Zustand bzw. bestimmungsgemäße Gebrauch der Hubvorrichtung zu verstehen, bei welchem diese bestimmungsgemäß in einem Isolator bzw. einer Produktionsanlage verbaut bzw. angeordnet ist.

Vorzugsweise ist im Bereich des ersten Endes der Hubstange eine Handhabungseinrichtung zur Handhabung von Objekten in dem Isolationsbereich angeordnet. Wie eingangs bereits erwähnt, dient die Handhabungseinrichtung zur Handhabung der Objekte und kann auf vorteilhafte Weise mittels der Hubvorrichtung verlagert werden. Derartige Objekte können Gegenstände wie insbesondere Vials, Flaschen etc. sein.

Die Hubvorrichtung ist dazu ausgebildet, im montierten Zustand durch eine Verlagerung der Hubstange das erste Ende der Hubstange in den Isolationsbereich hinein und wieder heraus zu bewegen. Unter Verlagerung der Hubstange ist im Zusammenhang mit der vorliegenden Erfindung eine Bewegung entlang der Längserstreckungsachse der Hubstange zu verstehen. Die Hubstange kann also, insbesondere nahezu vollständig, durch das Trennelement hindurch in den Isolationsbereich ein- und wieder ausgefahren werden.

Erfindungsgemäß ist nunmehr vorgesehen, dass die Hubvorrichtung einen Faltenbalg umfasst, der derart ausgebildet und angeordnet ist, dass er bei der Montage der Hubvorrichtung an dem Trennelement außerhalb des sterilen Isolationsbereichs anordenbar ist, und dass der Faltenbalg im vorgesehenen montierten Zustand einen sich bei Verlagerung der Hubstange aus dem Isolationsbereich in den Bedienbereich bewegenden Teil der Hubstange umschließt und von dem Bedienbereich trennt.

Erfindungsgemäß ist auch ein Isolator mit einem sterilen Isolationsbereich vorgesehen, wobei der Isolator ein Trennelement umfasst, welches insbesondere als Tischplatte ausgebildet ist. Der sterile Isolationsbereich ist dabei jedenfalls bereichsweise durch das Trennelement von einem nichtsterilen Bedienbereich getrennt. Der Isolator weist weiterhin eine Hubvorrichtung auf und zeichnet sich dadurch aus, dass die Hubvorrichtung gemäß Anspruch 1 ausgebildet ist. Die Hubvorrichtung ist derart an dem Trennelement montiert, dass der Faltenbalg außerhalb des sterilen Isolationsbereichs angeordnet ist, wobei der Faltenbalg einen sich bei Verlagerung der Hubstange aus dem Isolationsbereich in den Bedienbereich bewegenden Teil der Hubstange umschließt und von dem Bedienbereich trennt.

Im Falle des erfindungsgemäßen Isolators liegt also der montierte bzw. verbaute Zustand der Hubvorrichtung vor, also deren bestimmungsgemäßer Gebrauch bzw. Verwendung, im Rahmen dessen die Hubvorrichtung an bzw. in dem Isolator montiert ist.

Erfindungsgemäß ist ebenso eine Produktionsanlage mit einem Isolator vorgesehen, der erfindungsgemäß, wie vorstehend beschrieben, ausgebildet ist.

Die erfindungsgemäße Lösung bietet den Vorteil, dass die eingangs erörterten Problematiken im Wesentlichen vermieden werden. Dadurch, dass der Faltenbalg außerhalb des sterilen Isolationsbereichs anordenbar ist bzw. bei bestimmungsgemäßer Verwendung angeordnet ist, ergibt sich kein Platzproblem, wie es bei Anordnung des Faltenbalgs innerhalb des Isolators auftritt. Dennoch ist weiterhin eine sichere Entkopplung des sterilen von dem nichtsterilen Bereich gewährleistet. Aufgrund der vorteilhaften Anordnung außerhalb des Isolationsbereichs ist weiterhin gewährleistet, dass, wenn die Hülle des Faltenbalgs Undichtigkeiten aufweist, keine kontaminierte Luft angesaugt und in den Isolationsbereich hineingepresst, sondern gerade andersherum sterile Luft aus dem Inneren des Isolationsbereichs angesaugt und in den Außenbereich gepresst wird, insbesondere wenn hierzu passende Ventilanordnungen gewählt werden, sodass ein Einschleppen von Kontaminationen sicher vermieden ist.

Gemäß einer bevorzugten Weiterbildung ist vorgesehen, dass der Faltenbalg im vorgesehenen montierten Zustand an einer dem nichtsterilen Bedienbereich zugewandten Außenseite des Trennelements befestigbar bzw. befestigt ist. Die Außenseite ist insofern gegenüber dem nichtsterilen Bedienbereich exponiert. Hierdurch ergibt sich der Vorteil, dass der Faltenbalg bzw. die Vorrichtung mit vergleichsweise geringem Aufwand montierbar ist.

Insbesondere ist der Faltenbalg lösbar an den Trennelement befestigbar bzw. befestigt. Vorteilhafterweise ist dadurch eine beschädigungsfreie Demontage, beispielsweise zu Wartungszwecken, ermöglicht.

Bevorzugt ist die Hubstange um eine Rotationsachse, die der Achse der Längserstreckung der Hubstange entspricht, gegenüber dem Trennelement drehbar gelagert. Die Hubvorrichtung ist bevorzugt also vorteilhafterweise dazu ausgebildet, dass der Hubvorrichtung zugeordnete Objekt bzw. die Handhabungseinrichtung zusätzlich zu der Hubbewegung (vertikale Auf- und Abwärtsbewegung) auch durch eine Drehbewegung der Hubstange zu verlagern. In anderen Worten ist die Hubvorrichtung bevorzugt mit drehbarer Hubstange ausgebildet bzw. die Hubstange ist dazu in der Lage, Drehbewegungen auszuführen. Die Drehung kann durch einen entsprechend ausgebildeten und angeordneten Aktuator bewirkt werden.

Vorzugsweise ist der Faltenbalg gegenüber dem Trennelement drehbar gelagert und insbesondere gegenüber der Hubstange drehfest angeordnet. Bei diesem Ausführungsbeispiel dreht sich der Faltenbalg also bei Drehung der Hubstange mit und macht insofern die entsprechende Dreh- und Hubbewegung mit. Diese Ausführungsform kann beispielsweise über entsprechende Lagermittel am Trennelement bewirkt werden.

Alternativ dazu ist vorzugsweise der Faltenbalg gegenüber der Hubstange drehbar gelagert und insbesondere gegenüber dem Trennelement drehfest angeordnet. Bei diesem Ausführungsbeispiel dreht sich der Faltenbalg also bei Drehung der Hubstange nicht mit. Auch diese Ausführungsform kann beispielsweise über entsprechende Lagermittel gegenüber der Hubstange bewirkt werden. Insbesondere ist in diesem Fall also eine feste Verbindung des Faltenbalgs mit dem Trennelement vorgesehen.

Insofern kann insbesondere auch vorgesehen sein, dass der Faltenbalg sowohl gegenüber der Hubstange, als auch gegenüber dem Trennelement drehbar gelagert ist. In diesem Fall ist der Faltenbalg also unabhängig von der Drehbewegung der Hubstange eigenständig drehbar.

Die verschiedenen Ausführungsformen hinsichtlich der Drehbarkeit des Faltenbalgs resultieren in individuellen Vorteilen für den jeweiligen konkreten Anwendungsfall bzw. die Vorteile ergeben sich in Abhängigkeit der konkreten Anforderungen.

Erfindungsgemäß ist zwischen dem Faltenbalg und der Hubstange ein abgeschlossener Hohlraum ausgebildet, wobei weiter eine Spüleinrichtung vorgesehen ist, die einen Einlass zum Einbringen eines Dekontaminationsfluids in den Hohlraum umfasst und einen Auslass zum Abführen von Dekontaminationsfluid aus dem Hohlraum. Hierdurch ergibt sich der Vorteil, dass der Bereich zwischen Faltenbalg und Hubstange bei Bedarf dekontaminiert werden kann, sofern es wider Erwarten doch zu einer etwaigen Kontamination kommt. Vorteilhafterweise ist dadurch stets die Gewährleistung der Sterilität sichergestellt.

Die Spüleinrichtung umfasst erfindungsgemäß eine fluidische Verbindung mit dem vom Faltenbalg begrenzten Hohlraum.

Die Spüleinrichtung umfasst den Einlass und den Auslass, die jeweils eine fluidische Verbindung mit dem vom Faltenbalg begrenzten Hohlraum bilden. Bevorzugt weist die Spüleinrichtung weiterhin ein Reservoir, in welchem das Dekontaminationsfluid lagerbar bzw. gelagert ist, und ein Fluidleitungssystem zum Transport des Dekontaminationsfluids auf. Insofern sind der Einlass und der Auslass zumindest mit dem Reservoir und insbesondere auch mit dem Fluidleitungssystem fluidisch verbunden. Insbesondere handelt es sich bei dem Dekontaminationsfluid um Wasserstoffperoxid, welches in gasförmiger Form vorliegen kann.

Gemäß einer optionalen Weiterbildung weist die Spüleinrichtung zumindest ein Rückschlagventil zur Steuerung der Strömungsrichtung des Dekontaminationsfluids auf. Hierdurch ist es ermöglicht, dass das Spülen des Hohlraums mit dem Dekontaminationsfluid nicht aktiv, also mit einem speziell hierfür vorgesehenen Aktuator unter entsprechendem Energieaufwand, sondern passiv durch die ohnehin bei bestimmungsgemäßem Gebrauch bzw. im Normalbetrieb auftretende Hubbewegung der Hubstange und die damit einhergehende Ausdehnung und Komprimierung des Faltenbalgs, die in einer Pumpwirkung resultiert, erfolgen kann.

Der Faltenbalg fungiert in diesem Fall insofern als eine Art Pumpe. Im Falle einer Abwärtsbewegung der Hubstange, also eine Verlagerung des ersten Endes aus dem Isolationsbereich hinaus, wird der Faltenbalg langgezogen, wodurch ein Unterdruck entsteht, der eine Ansaugwirkung für das Dekontaminationsfluid bereitstellt. Bei der anschließenden Aufwärtsbewegung der Hubstange, also der Verlagerung des ersten Endes in den Isolationsbereich hinein, wird der Faltenbalg wiederum gestaucht und das zuvor angesaugte Dekontaminationsfluid aus dem Hohlraum hinaus gepresst bzw. ausgespült. Mittels des zumindest einen Rückschlagventils ist dabei ein nicht intendierter Rückfluss des Dekontaminationsfluids sicher vermieden bzw. das Dekontaminationsfluid ist bedingt durch das Rückschlagventil ausschließlich dazu in der Lage, gezielt in die gewünschte Strömungsrichtung zu strömen.

Insbesondere ist zwischen dem Einlass und dem Hohlraum ein erstes Rückschlagventil derart angeordnet, dass bei einer Verlagerung des ersten Endes der Hubstange von dem Isolationsbereich Weg, insbesondere in den Isolationsbereich hinein, dass Dekontaminationsfluid durch den Einlass durch das erste Rückschlagventil hindurch in den Hohlraum einströmt und durch das erste Rückschlagventil am Zurückströmen gehindert ist, und dass zwischen dem Hohlraum und dem Auslass ein zweites Rückschlagventil derart angeordnet ist, dass bei einer Verlagerung des ersten Endes der Hubstange zu dem Isolationsbereich hin, insbesondere in Isolationsbereich hinein, dass Dekontaminationsfluid von dem Hohlraum durch das zweite Rückschlagventil hindurch aus dem Auslass ausströmt und durch das zweite Rückschlagventil am Zurückströmen gehindert ist.

Durch diese Anordnung wird die zuvor beschriebene Spülfunktion und Kontrolle der Strömungsrichtung im Falle der durch den Faltenbalg bereitgestellten Pumpwirkung sicher gewährleistet.

Vorzugsweise ist zumindest ein Aktuator bzw. eine Fördereinrichtung, insbesondere Pumpe, zum Fördern des Dekontaminationsfluids vorgesehen. Hierdurch ist stets eine zuverlässige Förderung des Dekontaminationsfluids und damit eine zuverlässige Dekontamination sichergestellt. Bevorzugt ist die Fördereinrichtung zusätzlich zu der vorstehend bereits beschriebenen durch den Faltenbalg bereitgestellten Pumpwirkung vorhanden, sodass auch für den Fall, dass die Pumpwirkung durch den Faltenbalg alleine nicht ausreichen sollte, eine zuverlässige Dekontamination ermöglicht ist.

Alternativ kann die Fördereinrichtung die Pumpwirkung des Faltenbalgs auch ersetzen, sodass eine Förderung unabhängig vom Faltenbalg und dem zumindest einen Rückschlagventil, welches jedoch dennoch vorhanden sein kann, erfolgen kann. Vorteilhafterweise ist durch die Fördereinrichtung die Spülung des Hohlraums auch unabhängig von der Hubbewegung der Hubstange und insofern des Betriebs der Hubvorrichtung ermöglicht.

Bevorzugt ist vorgesehen, dass der Einlass der Spüleinrichtung fluidisch mit dem sterilen Isolationsbereich gekoppelt sind. Es kann auch der Auslass der Spüleinrichtung fluidisch mit dem sterilen Isolationsbereich gekoppelt sein. Das Dekontaminationsfluid, insbesondere Wasserstoffperoxid, kann auch nach durchströmen des Hohlraums ausreichend potent sein, um für weitere Dekontaminationen verwendet zu werden. Der Auslass kann aber auch das Dekontaminationsfluid abführen, insbesondere einer Entsorgung zuführen, bspw. einem Auffangreservoir. Insofern stellt in diesem Fall der sterile Isolationsbereich bzw. der Isolator das zuvor erwähnte Reservoir für das Dekontaminationsfluid dar. In diesem Fall kann das zur standardmäßigen Dekontamination des Isolators, wie sie im Normalfall in regelmäßigen Abständen stattfindet, verwendete Dekontaminationsfluid auch zu Dekontamination des Hohlraums genutzt werden, da es einfach durch den Einlass aus dem Isolator auch in den Hohlraum einströmt bzw. eingeleitet wird.

Die Aufgabe wird, wie oben erwähnt, auch gelöst durch ein Verfahren zur Dekontamination einer Hubvorrichtung, eines Isolators oder einer Produktionsanlage, wie vorstehend beschrieben.

Bei dem Verfahren ist vorgesehen, dass das Dekontaminationsfluid aus einem mit dem Einlass und dem Auslass fluidisch gekoppelten Reservoir, insbesondere dem Isolator, in den Hohlraum gefördert wird, und dass das Fördern des Dekontaminationsfluids aktiv durch eine Fördereinrichtung bzw. einen Aktuator oder passiv durch eine Hubbewegung der Hubstange und des Faltenbalgs erfolgt.

Für genauere Details hinsichtlich der konkreten Funktionsweise und der entsprechenden Vorteile wird auf das vorstehend Beschriebene verwiesen.

Im Folgenden soll die Erfindung anhand der Figuren näher erläutert werden. Gleiche Elemente sind mit denselben Bezugszeichen versehen, gegebenenfalls lediglich einmal. Es zeigen:
- Figur 1: eine an einem Isolator montierte Hubvorrichtung in einem ersten Zustand;
- Figur 2: die Hubvorrichtung aus Figur 1 in einem zweiten Zustand;
- Figur 3: die Hubvorrichtung gemäß einem ersten Ausführungsbeispiel; und
- Figur 4: die Hubvorrichtung gemäß einem zweiten Ausführungsbeispiel.

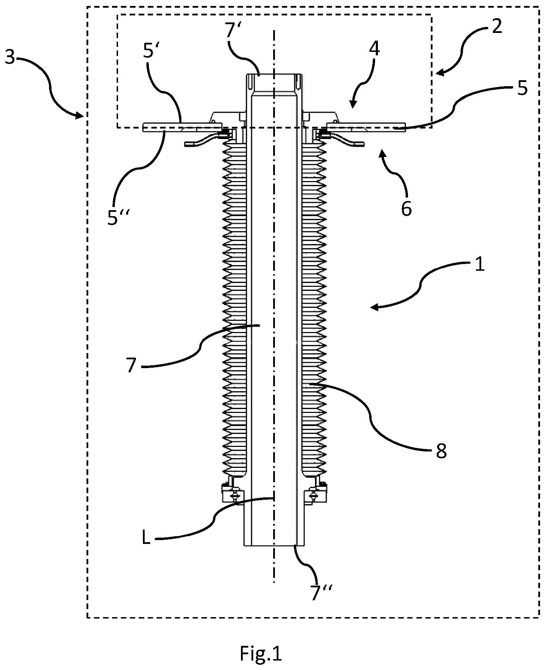

Figur 1 zeigt in einer vereinfachten Darstellung eine Hubvorrichtung 1. Die Hubvorrichtung 1 ist zur Anordnung bzw. Montage an oder in einem vorliegend aus Übersichtlichkeitsgründen nur anhand von gestrichelten Linien angedeuteten Isolator 2 vorgesehen bzw. konzipiert. Der Isolator 2 ist Teil einer ebenfalls nur schematisch durch ein gestrichelt gezeichnetes Rechteck dargestellten Produktionsanlage 3.

Der Isolator 2 ist derart konzipiert bzw. ausgebildet, dass er eine sterile Handhabung von Produkten, beispielsweise pharmazeutischen oder medizintechnischen Erzeugnissen, ermöglicht. Zu diesem Zweck bildet der Isolator 2 einen sterilen Isolationsbereich 4 aus, der zumindest durch ein Trennelement 5 von einem nicht sterilen Bedienbereich 6 getrennt ist. Vorliegend handelt es sich bei dem Trennelement 5 um eine Tischplatte. Insofern weist der Isolator 2 zumindest das Trennelement 5 auf, um den sterilen Isolationsbereich 4 von den nicht sterilen Bedienbereich 6 zu trennen.

Selbstverständlich kann der Isolator 2 noch weitere, bei bekannten gattungsgemäßen Isolatoren vorgesehene Komponenten, beispielsweise eine Handhabungseinrichtung, ein Transfersystem oder dergleichen, aufweisen, die vorliegend aus Übersichtlichkeitsgründen jedoch nicht gezeigt sind.

Figur 1 zeigt die Hubvorrichtung 1 bei bestimmungsgemäßem Gebrauch bzw. im montierten/verbauten Zustand, also jenem Zustand, bei welchem die Hubvorrichtung 1 an bzw. in dem Isolator 2 verbaut ist.

Im montierten Zustand ist die Hubvorrichtung 1 im Bereich des Trennelements 5 angeordnet. Die Hubvorrichtung 1 weist eine Hubstange 7 mit einem ersten Ende 7' und einen zweiten Ende 7" auf und ist dazu ausgebildet, im in Figur 1 gezeigten montierten Zustand durch eine Verlagerung der Hubstange 7 das erste Ende 7' in den Isolationsbereich 4 hinein und wieder heraus zu bewegen, also eine Hubbewegung auszuführen. Unter Hubbewegung ist insofern eine vertikale Auf- und Abwärtsbewegung bzw. eine Bewegung entlang einer Längserstreckungsachse L der Hubstange 7 zu verstehen.

Die Hubvorrichtung 1 dient dazu, durch die Verlagerung der Hubstange 7 bzw. die Hubbewegung in dem Isolationsbereich 4 angeordnete und der Hubvorrichtung 1 zugeordnete Objekte oder Vorrichtungen zu verlagern. Die Hubvorrichtung 1 ist hierzu bei bestimmungsgemäßem Gebrauch, wie in Figur 1 gezeigt, derart an dem Trennelement 5 montiert, dass die Hubstange 7 bzw. deren erstes Ende 7' durch eine Öffnung des Trennelements 5 durch das Trennelement 5 hindurch verlagerbar ist. In anderen Worten wird im Normalbetrieb der Hubvorrichtung 1 bzw. des Isolators 2 oder der Produktionsanlage 3 die Hubstange 7 regelmäßig zumindest bereichsweise durch das Trennelement 5 hindurch aus dem Isolationsbereich 4 in den Bedienbereich 6 hinaus und wieder hinein verlagert.

Insbesondere ist an dem ersten Ende 7' der Hubstange 7 eine vorliegend aus Übersichtlichkeitsgründen nicht gezeigte Handhabungseinrichtung angeordnet bzw. montiert, die zur Handhabung von Objekten innerhalb des Isolationsbereichs 4 ausgebildet und insofern durch die Hubvorrichtung 1 verlagerbar ist.

Um bei der regelmäßig ausgeführten Hubbewegung ein Einschleppen von Kontaminationen in den Isolationsbereich 4 und damit eine Beeinträchtigung der Sterilität innerhalb des Isolators 2 zu vermeiden, ist vorliegend vorgesehen, dass die Hubvorrichtung 1 einen Faltenbalg 8 umfasst, der den sich aus dem Isolationsbereich 4 bewegenden Teil der Hubstange 7 umschließt und von dem Bedienbereich 6 trennt.

Vorteilhafterweise ist bei der vorliegenden Erfindung vorgesehen, dass der Faltenbalg 8 derart ausgebildet und angeordnet ist, dass er bei der Montage der Hubvorrichtung 1 an dem Isolator 2 bzw. dessen Trennelement 5 außerhalb des sterilen Isolationsbereichs 4 anordenbar bzw. bei dem in Figur 1 gezeigten montierten Zustand, wie dort gut zu erkennen ist, angeordnet ist. Vorliegend ist der Faltenbalg 8 dabei an einer dem nicht sterilen Bedienbereich 6 zugewandten Außenseite 5' des Trennelements 5 befestigt. Das Trennelement 5 weist insofern die gegenüber dem Bedienbereich exponierte Außenseite 5' sowie eine gegenüber dem sterilen Isolationsbereich 4 exponierte Innenseite 5" auf.

Insbesondere ist der Faltenbalg 8 an den Trennelement 5 lösbar befestigt, beispielsweise kraft- oder formschlüssig, um bei Bedarf eine zerstörungsfreie Demontage, beispielsweise zu Wartungszwecken, zu ermöglichen. Alternativ kann der Faltenbalg 8 auch nach erstmaliger Montage unlösbar, beispielsweise stoffschlüssig, befestigt werden, insbesondere um eine besonders zuverlässige Dichtigkeit zu gewährleisten.

Vorzugsweise ist vorgesehen, dass die Hubvorrichtung 1 mit einer drehbaren Hubstange 7 ausgebildet ist, sodass durch die Hubvorrichtung 1 neben der Hubbewegung auch eine Drehbewegung ermöglicht ist. Insofern ist vorzugsweise die Hubstange um eine Rotationsachse, die der Längserstreckungsachse L der Hubstange 7 entspricht, gegenüber dem Trennelement 5 drehbar gelagert.

Die drehbare Lagerung kann dabei durch vorliegend aus Übersichtlichkeitsgründen nicht gezeigte Lagermittel bewirkt werden. Je nach konkretem Anwendungsfall kann zusätzlich vorgesehen sein, dass auch der Faltenbalg 8 drehbar gelagert ist, beispielsweise ebenfalls durch entsprechende Lagermittel. Die Drehbarkeit des Faltenbalgs 8 in Bezug auf das Trennelement 5 und/oder die Hubstange 7 kann sich dabei in Abhängigkeit der konkreten Anforderungen an die Hubvorrichtung 1 unterscheiden.

Diesbezüglich ist gemäß einer ersten Ausführungsform der Faltenbalg 8 gegenüber dem Trennelement 5 drehbar gelagert und gegenüber der Hubstange 7 drehfest angeordnet, sodass sich der Faltenbalg 8 bei Drehung der Hubstange 7 mitdreht und die entsprechende Dreh- und Hubbewegung mitmacht.

Bei einer zweiten Ausführungsform hingegen ist der Faltenbalg 8 gegenüber der Hubstange 7 drehbar gelagert und gegenüber dem Trennelement 5 drehfest angeordnet, sodass sich der Faltenbalg 8 bei Drehung der Hubstange 7 nicht mitdreht.

Gemäß einer dritten Ausführungsform kann der Faltenbalg 8 aber auch sowohl gegenüber dem Trennelement 5, als auch gegenüber der Hubstange 7 drehbar gelagert sein, sodass er unabhängig von der Drehbewegung der Hubstange 7, also eigenständig, drehbar ist.

Figur 1 zeigt die Hubvorrichtung 1 in einem Zustand, bei welchem die Hubstange 7 größtenteils aus dem Isolator 2 herausgefahren ist, sodass der die Hubstange 7 umschließende Faltenbalg 8 sich in einem langgezogenen bzw. gestreckten Zustand befindet. Fährt die Hubstange 7 wieder in Isolationsbereich 4 hinein, so wird der Faltenbalg 8 komprimiert.

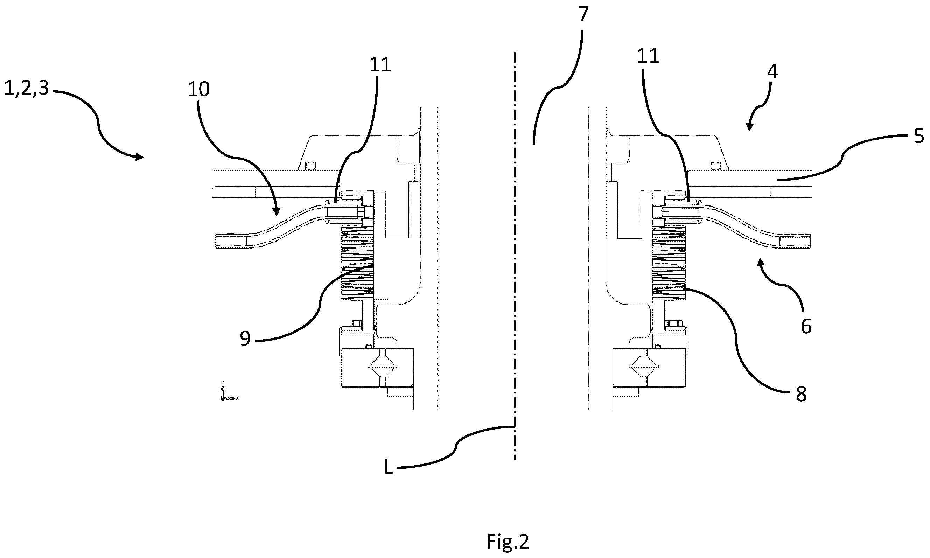

Figur 2 zeigt einen vergrößerten Ausschnitt der zuvor anhand von Figur 1 beschriebenen Hubvorrichtung 1. Bei der Darstellung gemäß Figur 2 befindet sich der Faltenbalg 8 im zuvor erwähnten komprimierten Zustand, als in jenem Zustand, in welchem die Hubstange 7 zumindest weitestgehend in den Isolationsbereich 4 hinein verlagert ist.

Wie der in Figur 2 gezeigten Detailansicht zu entnehmen ist, ist zwischen dem Faltenbalg 8 und der Hubstange 7 ein abgeschlossener Hohlraum 9 ausgebildet. Weist der Faltenbalg 8 Undichtigkeiten auf, so kann es dazu kommen, dass Kontaminationen in den Hohlraum 9 eindringen und sich an der Hubstange 7 anlagern. Aufgrund der vorteilhaften Anordnung des Faltenbalgs 8 außerhalb des Isolationsbereichs 4 werden diese Kontaminationen jedoch nicht bei der Verlagerung der Hubstange 7 in den Isolationsbereich 4 in diesen eingeschleppt, sondern bei der mit Verlagerung einhergehenden Komprimierung des Faltenbalgs 8 und des dabei erzeugten Überdrucks wieder aus dem Hohlraum 9 in die Umgebung ausgeblasen. Zwischen Hubstange 7 und Trennelement 5 kann ein Dichtelement angebracht sein, welches möglichst potentiell vorhandene Kontaminationen an der Hubstange 7, welche außerhalb des Isolationsbereichs 4 im Hohlraum 9 vorhanden sein können, vom Eindringen in den Isolationsbereichs 4 abhält. Das Dichtelement kann dabei eine quasi abstreifende Wirkung bei Bewegung der Hubstange 7 haben bzw. als eine Art Abstreifer fungieren.

Jedoch kann es vorkommen, dass Kontaminationen nicht alleine durch Überdruck entfernbar sind. Für diesen Fall aber auch generell ist es zweckmäßig bzw. vorteilhaft, den Hohlraum 9 bei Bedarf anderweitig dekontaminieren zu können.

Zu diesem Zweck ist vorliegend vorteilhafterweise eine in Figur 2 aus Übersichtlichkeitsgründen nur angedeutete Spüleinrichtung 10 vorgesehen, mittels welcher ein Einbringen eines Dekontaminationsfluids, insbesondere gasförmiges Wasserstoffperoxid, in den Hohlraum 9 und ein anschließendes Abführen des Dekontaminationsfluids ermöglicht ist. Die Spüleinrichtung 10 ist, wie in Figur 2 gezeigt, mittels zumindest 2 Anschlussvorrichtungen 11 fluidisch mit dem Hohlraum 9 verbunden, sodass der Hohlraum 9 mit dem Dekontaminationsfluid spülbar ist.

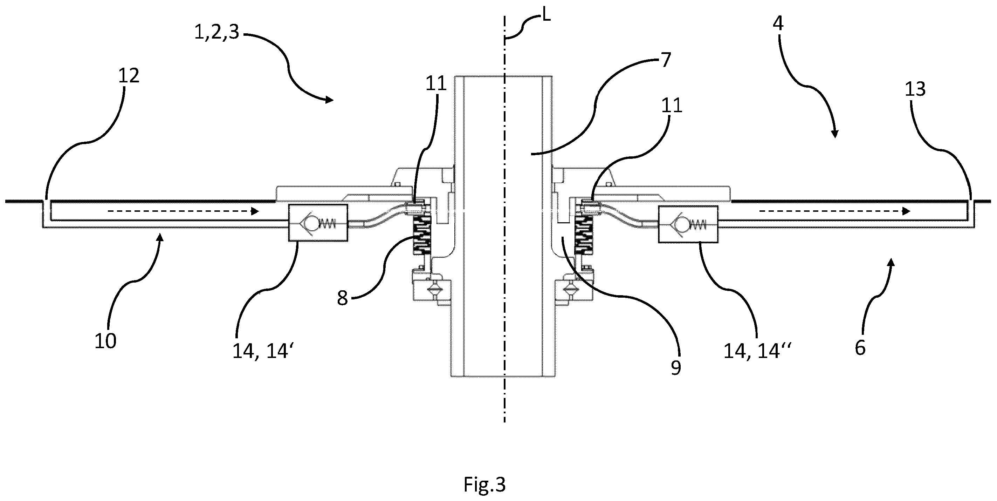

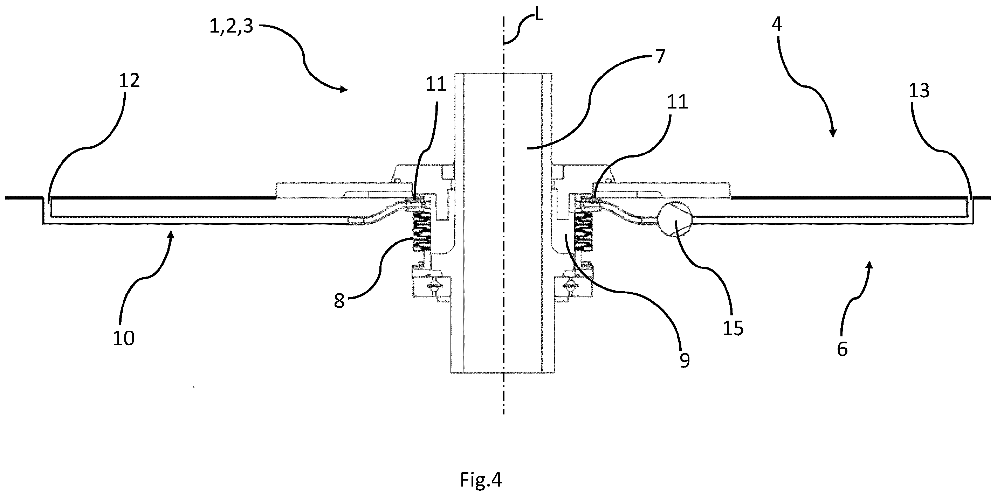

Die Spüleinrichtung 10 soll im Folgenden anhand der Figuren 3 und 4 näher erläutert werden. Figur 3 zeigt die Spüleinrichtung 10 gemäß einem ersten Ausführungsbeispiel, Figur 4 gemäß einem zweiten Ausführungsbeispiel.

Die Spüleinrichtung 10 weist zumindest einen Einlass 12 zum Einbringen des Dekontaminationsfluids und einen Auslass 13 zum Abführen des Dekontaminationsfluids auf. Vorliegend weist die Spüleinrichtung 10 zudem ein nur beispielhaft angedeutetes und insofern nicht näher beziffertes Fluidleitungssystem auf, mittels welchem der Einlass 12 und der Auslass 13 über die Anschlussvorrichtungen 11 mit dem Hohlraum 9 fluidisch gekoppelt sind.

Vorliegend sind der Einlass 12 und der Auslass 13 fluidisch mit dem Isolator 2 bzw. dem Isolationsbereich 4 gekoppelt. Standartmäßig wird im Normalbetrieb des Isolators 2 der Isolationsbereich 4 in regelmäßigen Abständen mit einem Dekontaminationsfluid dekontaminiert bzw. gespült. Durch die fluidische Kopplung der Spüleinrichtung 10 mit dem Isolator 2 bzw. Isolationsbereich 4 kann das zur Dekontamination des Isolationsbereichs 4 genutzte Dekontaminationsfluid vorteilhafterweise gleichzeitig auch zur Dekontamination des Hohlraums 9 genutzt werden.

Insofern dient vorliegend der Isolator 2 bzw. der Isolationsbereich 4 als Reservoir für das Dekontaminationsfluid. Selbstverständlich kann alternativ auch ein separates, speziell hierfür vorgesehenes und eingerichtetes Reservoir vorgesehen sein, das mit der Spüleinrichtung 10 fluidisch gekoppelt ist.

Gemäß dem in Figur 3 gezeigten ersten Ausführungsbeispiel weist die Spüleinrichtung 10 weiterhin zumindest ein, vorliegend zwei, Rückschlagventile 14 auf, mittels welchem die Strömungsrichtung des Dekontaminationsfluids vorgebbar bzw. steuerbar ist. Hierzu ist ein erstes Rückschlagventil 14' zwischen dem Einlass 12 und dem Hohlraum 9 und ein zweites Rückschlagventil 14" zwischen dem Hohlraum 9 und dem Auslass 13 angeordnet.

Bei dem ersten Ausführungsbeispiel der Spüleinrichtung 10 ist bevorzugt vorgesehen, dass das Fördern des Dekontaminationsfluids in den Hohlraum 9 hinein und wieder heraus, also das Spülen, passiv erfolgt. Passiv bedeutet in diesem Zusammenhang, dass das Fördern des Dekontaminationsfluids ohne eine speziell hierfür vorgesehene und angeordnete Fördereinrichtung, beispielsweise ein Aktuator wie etwa eine Pumpe, und entsprechend ohne zusätzlichen, zum Fördern benötigten, Energieaufwand erfolgt.

Stattdessen wird bei dem ersten Ausführungsbeispiel die ohnehin bei der mit der Hubbewegung der Hubstange 7 erzeugte, aus der mit der Hubbewegung einhergehenden Komprimierung und Ausdehnung des Faltenbalgs 8 resultierende, Pumpwirkung zur Förderung des Dekontaminationsfluids genutzt.

Wird die Hubstange 7 aus dem Isolationsbereich 4 heraus bewegt, so streckt sich der Faltenbalg 8 und in dem Hohlraum 9 wird ein Unterdruck erzeugt. Hierdurch wird Dekontaminationsfluid aus dem Isolationsbereich 4 durch den Einlass 12 in den Hohlraum 9 hinein angesaugt bzw. gefördert, wie in Figur 3 durch einen gestrichelt gezeichneten ersten Pfeil beispielhaft dargestellt ist. Mittels des ersten Rückschlagventils 14' ist dabei ein nicht gewünschter Rückfluss bzw. ein Zurückströmen des angesaugten Dekontaminationsfluids sicher vermieden. Wird die Hubstange 7 im Anschluss wieder in den Isolationsbereich 4 hinein bewegt, so wird der Faltenbalg 8 und damit der Hohlraum 9 komprimiert und das zuvor durch den Unterdruck angesaugte Dekontaminationsfluid aus dem Hohlraum 9 herausgedrückt, wie in Figur 3 durch einen gestrichelt gezeichneten zweiten Pfeil beispielhaft dargestellt ist. Da das Dekontaminationsfluid an dieser Stelle durch das erste Rückschlagventil 14' am zurückströmen in Richtung des Einlass 12 gehindert ist, kann es nur durch das zweite Rückschlagventil 14" hindurch in Richtung des Auslass 13 aus dem Hohlraum 9 entweichen. Durch das zweite Rückschlagventil 14" ist wiederum sicher vermieden, dass einmal aus dem Hohlraum 9 ausgeströmtes bzw. herausgedrücktes Dekontaminationsfluid wieder in diesen zurückströmen kann. Vielmehr wird das Dekontaminationsfluid durch den Auslass 13 im vorliegenden Beispiel wieder in den Isolationsbereich 4 abgegeben, dort kann es bspw. in einem Auffangbehälter gesammelt werden oder kann dort im Rahmen der standardmäßigen Dekontamination des Isolationsbereichs 14 fachgerecht entsorgt werden. Der Auslass kann auch derart angeordnet sein, dass das Dekontaminationsfluid direkt einer Entsorgung zuführt. Diese Entsorgung kann bspw. durch einen Auffangbehälter realisiert sein.

Die beiden in Figur 3 gezeigten Pfeile stellen insofern die Förder- bzw. Strömungsrichtung des Dekontaminationsfluids dar.

Im Falle des in Figur 3 gezeigten ersten Ausführungsbeispiels erfolgt die Dekontamination des Hohlraums 9 also passiv durch die intrinsisch vorhandene Saug- bzw. Pumpwirkung des Faltenbalgs 8. Sollte jedoch diese Pumpwirkung alleine nicht für eine ausreichende Förderung von Dekontaminationsfluid ausreichen, so kann vorgesehen sein, dass zusätzlich eine separate Fördereinrichtung, beispielsweise eine Pumpe, vorhanden ist, die das Dekontaminationsfluid bei Bedarf in den Hohlraum 9 fördert und insofern die Pumpwirkung des Faltenbalgs 8 unterstützt.

Figur 4 zeigt die Spüleinrichtung 10 gemäß einem zweiten Ausführungsbeispiel. Bezüglich gleicher Elemente wird auf das vorstehend Beschriebene verwiesen und im Folgenden nur die Unterschiede erörtert.

Das in Figur 4 gezeigte zweite Ausführungsbeispiel unterscheidet sich von dem zuvor erörterten ersten Ausführungsbeispiel dadurch, dass die Förderung des Dekontaminationsfluids bevorzugt nunmehr nicht mehr passiv durch die Pumpwirkung des Faltenbalgs 8, sondern aktiv unter Energieaufwendung mittels einer Fördereinrichtung 15 erfolgt. Die Fördereinrichtung 15 ist insofern ein Teil der Spüleinrichtung 10. Die Fördereinrichtung 15 ist derart angeordnet und fluidisch mit dem Einlass 12, dem Hohlraum 9 und dem Auslass 13 verbunden, dass mittels der von der Fördereinrichtung 15 erzeugbaren Förderwirkung das Dekontaminationsfluid durch den Einlass 12 in den Hohlraum 9 hinein und durch den Auslass 13 wieder hinaus gefördert wird. Die Fördereinrichtung 15 ist also ausgebildet und angeordnet, um den Hohlraum 9 mit dem Dekontaminationsfluid durchzuspülen.

Bei dem zweiten Ausführungsbeispiel sind die Rückschlagventile 14 aufgrund der Förderwirkung der Fördereinrichtung 15 nicht zwingend notwendig, können jedoch optional zusätzlich vorgesehen sein. Das zweite Ausführungsbeispiel mit der Fördereinrichtung 15 bietet den Vorteil, dass stets ein zuverlässiges Fördern des Dekontaminationsfluids ermöglicht ist, insbesondere unabhängig von der Hubbewegung der Hubstange 7 bzw. des Faltenbalgs 8, also unabhängig von einem Betrieb der Hubvorrichtung 1.

Zusammenfassend bietet die vorstehend erörterte Erfindung den Vorteil, dass ein Einschleppen von Kontaminationen durch die Hubvorrichtung 1 in den Isolationsbereich 4 sicher vermeidbar ist.

## Patentansprüche

1. Hubvorrichtung (1) zur Verwendung in einem sterilen Isolationsbereich (4), wobei der sterile Isolationsbereich (4) jedenfalls bereichsweise durch ein Trennelement (5), das insbesondere als Tischplatte ausgebildet ist, von einem nichtsterilen Bedienbereich (6)getrennt ist, wobei die Hubvorrichtung (1) mindestens eine Hubstange (7) mit einem ersten Ende (7'), das im montierten Zustand zur Anordnung in dem sterilen Isolationsbereich (4) vorgesehen ist, und einem zweiten Ende (7"), das im montierten Zustand zur Anordnung außerhalb des Isolationsbereichs (4) vorgesehen ist, aufweist, insbesondere wobei im Bereich des ersten Endes (7') der Hubstange (7) eine Handhabungseinrichtung zur Handhabung von Objekten in dem Isolationsbereich (4) angeordnet ist, und wobei die Hubvorrichtung (1) ausgebildet ist, um im montierten Zustand durch eine Verlagerung der Hubstange (7) das erste Ende (7') der Hubstange (7) im dem Isolationsbereich (4) zu bewegen, wobei die Hubvorrichtung (1) einen Faltenbalg (8) umfasst, der derart ausgebildet und angeordnet ist, dass er bei der Montage der Hubvorrichtung (1) an dem Trennelement (5) außerhalb des sterilen Isolationsbereichs (4) anordenbar ist, und dass der Faltenbalg (8) im vorgesehenen montierten Zustand einen sich bei Verlagerung der Hubstange (7) aus dem Isolationsbereich (4) in den Bedienbereich (6) bewegenden Teil der Hubstange (7) umschließt und von dem Bedienbereich (6) trennt, **dadurch gekennzeichnet, dass** zwischen dem Faltenbalg (8) und der Hubstange (7) ein abgeschlossener Hohlraum (9) ausgebildet ist, wobei weiter eine Spüleinrichtung (10) vorgesehen ist, die einen Einlass (12) zum Einbringen eines Dekontaminationsfluids, insbesondere Wasserstoffperoxid, in den Hohlraum (9) umfasst und einen Auslass (13) zum Abführen von Dekontaminationsfluid aus dem Hohlraum (9).

2. Isolator (2) mit einem sterilen Isolationsbereich (4),
wobei der Isolator (2) ein Trennelement (5) umfasst, wobei der sterile Isolationsbereich (4) jedenfalls bereichsweise durch das Trennelement (5), das insbesondere als Tischplatte ausgebildet ist, von einem nichtsterilen Bedienbereich (6)getrennt ist, und wobei der Isolator (2) eine Hubvorrichtung (1) umfasst, **dadurch gekennzeichnet, dass** die Hubvorrichtung (1) nach Anspruch 1 ausgebildet ist und derart an dem Trennelement (5) montiert ist, dass der Faltenbalg (8) außerhalb des sterilen Isolationsbereichs (4) angeordnet ist, und dass der Faltenbalg (8) einen sich bei Verlagerung der Hubstange (7) aus dem Isolationsbereich (4) in den Bedienbereich (6) bewegenden Teil der Hubstange (7) umschließt und von dem Bedienbereich (6) trennt.

3. Produktionsanlage (3) mit einem Isolator (2) nach Anspruch 2.

4. Hubvorrichtung, Isolator oder Produktionsanlage nach einem der vorhergehenden Ansprüche, **dadurch gekennzeichnet, dass** der Faltenbalg (8) im vorgesehenen montierten Zustand an einer dem nichtsterilen Bedienbereich (6) zugewandten Außenseite (5') des Trennelements (5), insbesondere lösbar, befestigt ist.

5. Hubvorrichtung, Isolator oder Produktionsanlage nach einem der vorhergehenden Ansprüche, **dadurch gekennzeichnet, dass** die Hubstange (7) um eine Rotationsachse, die der Achse der Längserstreckung der Hubstange (7) entspricht, gegenüber dem Trennelement (5) drehbar gelagert ist.

6. Hubvorrichtung, Isolator oder Produktionsanlage nach Anspruch 5, **dadurch gekennzeichnet, dass** der Faltenbalg (8) gegenüber dem Trennelement (5) drehbar gelagert ist und insbesondere gegenüber der Hubstange (7) drehfest angeordnet ist.

7. Hubvorrichtung, Isolator oder Produktionsanlage nach Anspruch 5, **dadurch gekennzeichnet, dass** der Faltenbalg (8) gegenüber der Hubstange (7) drehbar gelagert ist und insbesondere gegenüber dem Trennelement (5) drehfest angeordnet ist.

8. Hubvorrichtung, Isolator oder Produktionsanlage nach einem der vorhergehenden Ansprüche, **dadurch gekennzeichnet, dass** das Spüleinrichtung (10) zumindest ein Rückschlagventil (14) zur Steuerung der Strömungsrichtung des Dekontaminationsfluids aufweist.

9. Hubvorrichtung, Isolator oder Produktionsanlage nach einem der vorhergehenden Ansprüche, **dadurch gekennzeichnet, dass** zwischen dem Einlass (12) und dem Hohlraum (9) ein erstes Rückschlagventil (14') derart angeordnet ist, dass bei einer Verlagerung des ersten Endes (7') der Hubstange (7) von dem Isolationsbereich (4) weg, insbesondere in den Isolationsbereich (4) hinein, das Dekontaminationsfluid durch den Einlass (12) durch das erste Rückschlagventil (14') hindurch in den Hohlraum (9) einströmt und durch das erste Rückschlagventil (14') am Zurückströmen gehindert ist, und dass zwischen dem Hohlraum (9) und dem Auslass (13) ein zweites Rückschlagventil (14") derart angeordnet ist, dass bei einer Verlagerung des ersten Endes (7') der Hubstange (7) zu dem Isolationsbereich (4) hin, insbesondere in den Isolationsbereich (4) hinein, das Dekontaminationsfluid von dem Hohlraum (9) durch das zweite Rückschlagventil (14") hindurch aus dem Auslass (13) ausströmt und durch das zweite Rückschlagventil (14") am Zurückströmen gehindert ist.

10. Hubvorrichtung, Isolator oder Produktionsanlage nach einem der vorhergehenden Ansprüche, **dadurch gekennzeichnet, dass** zumindest eine Fördereinrichtung (15), insbesondere Pumpe, zum Fördern des Dekontaminationsfluids vorgesehen ist.

11. Hubvorrichtung, Isolator oder Produktionsanlage nach einem der vorhergehenden Ansprüche, **dadurch gekennzeichnet, dass** der Einlass (12) und der Auslass (13) fluidisch mit dem sterilen Isolationsbereich (4) gekoppelt sind.

12. Verfahren zur Dekontamination einer Hubvorrichtung (1), eines Isolators (2) oder einer Produktionsanlage (3) nach einem der vorhergehenden Ansprüche, **dadurch gekennzeichnet, dass**
- das Dekontaminationsfluid aus einem mit dem Einlass (12) und dem Auslass (13) fluidisch gekoppelten Reservoir, insbesondere dem sterilen Isolationsbereich (4), in den Hohlraum (9) gefördert wird, und
- das Fördern des Dekontaminationsfluids aktiv durch eine Fördereinrichtung (15) oder passiv durch eine Hubbewegung der Hubstange (7) und des Faltenbalgs (8) erfolgt.

## Claims

1. Lifting device (1) for use in a sterile isolation region (4), the sterile isolation region (4) being separated from a non-sterile operating region (6) at least in part by a separating element (5), which is designed in particular as a table top, the lifting device (1) having at least one lifting rod (7) with a first end (7') which, in the assembled state, is provided for arrangement in the sterile isolation region (4), and a second end (7") which, in the assembled state, is provided for arrangement outside the isolation region (4), a handling apparatus for handling objects in the isolation region (4) in particular being arranged in the region of the first end (7') of the lifting rod (7), and the lifting device (1) being designed, in the assembled state, to move the first end (7') of the lifting rod (7) in the isolation region (4) by displacing the lifting rod (7), the lifting device (1) comprising a bellows (8) which is designed and arranged in such a way that during assembly of the lifting device (1) said bellows can be arranged on the separating element (5) outside the sterile isolation region (4), and that the bellows (8), in the intended assembled state, encloses a part of the lifting rod (7) which moves when the lifting rod (7) is displaced from the isolation region (4) into the operating region (6), and separates said part from the operating region (6),
**characterized in that** a closed cavity (9) is formed between the bellows (8) and the lifting rod (7), a flushing apparatus (10) further being provided, comprising an inlet (12) for introducing a decontamination fluid, in particular hydrogen peroxide, into the cavity (9) and an outlet (13) for discharging decontamination fluid from the cavity (9).

2. Isolator (2) having a sterile isolation region (4),
the isolator (2) comprising a separating element (5), the sterile isolation region (4) being separated from a non-sterile operating region (6) at least in part by the separating element (5), which is designed in particular as a table top, and the isolator (2) comprising a lifting device (1), **characterized in that** the lifting device (1) is designed according to claim 1 and is mounted on the separating element (5) in such a way that the bellows (8) is arranged outside the sterile isolation region (4), and that the bellows (8) encloses a part of the lifting rod (7) which moves when the lifting rod (7) is displaced from the isolation region (4) into the operating region (6), and separates said part from the operating region (6).

3. Production plant (3) having an isolator (2) according to claim 2.

4. Lifting device, isolator or production plant according to any of the preceding claims, **characterized in that** the bellows (8), in the intended assembled state, is fastened, in particular detachably, to an outer face (5') of the separating element (5) facing the non-sterile operating region (6).

5. Lifting device, isolator or production plant according to any of the preceding claims, **characterized in that** the lifting rod (7) is mounted so as to be rotatable relative to the separating element (5) about an axis of rotation which corresponds to the axis of the longitudinal extension of the lifting rod (7).

6. Lifting device, isolator or production plant according to claim 5, **characterized in that** the bellows (8) is rotatably mounted relative to the separating element (5) and is in particular mounted for conjoint rotation with the lifting rod (7).

7. Lifting device, isolator or production plant according to claim 5, **characterized in that** the bellows (8) is rotatably mounted relative to the lifting rod (7) and is in particular mounted for conjoint rotation with the separating element (5).

8. Lifting device, isolator or production plant according to any of the preceding claims, **characterized in that** the flushing apparatus (10) has at least one check valve (14) for controlling the flow direction of the decontamination fluid.

9. Lifting device, isolator or production plant according to any of the preceding claims, **characterized in that** a first check valve (14') is arranged between the inlet (12) and the cavity (9) in such a way that, when the first end (7') of the lifting rod (7) is displaced away from the isolation region (4), in particular into the isolation region (4), the decontamination fluid flows through the inlet (12) and through the first check valve (14') into the cavity (9) and is prevented from flowing back by the first check valve (14'), and **in that** a second check valve (14") is arranged between the cavity (9) and the outlet (13) in such a way that, when the first end (7') of the lifting rod (7) is displaced toward the isolation region (4), in particular into the isolation region (4), the decontamination fluid flows from the cavity (9) through the second check valve (14") out of the outlet (13) and is prevented from flowing back by the second check valve (14").

10. Lifting device, isolator or production plant according to any of the preceding claims, **characterized in that** at least one conveying apparatus (15), in particular a pump, is provided for conveying the decontamination fluid.

11. Lifting device, isolator or production plant according to any of the preceding claims, **characterized in that** the inlet (12) and the outlet (13) are fluidically coupled to the sterile isolation region (4).

12. Method for decontamination of a lifting device (1), an isolator (2) or a production plant (3) according to any of the preceding claims, **characterized in that**
- the decontamination fluid is conveyed into the cavity (9) from a reservoir, in particular the sterile isolation region (4), fluidically coupled to the inlet (12) and the outlet (13) and
- the decontamination fluid is conveyed actively by a conveying apparatus (15) or passively by a lifting movement of the lifting rod (7) and the bellows (8).

## Revendications

1. Dispositif de levage (1) destiné à être utilisé dans une zone d'isolation (4) stérile, dans lequel la zone d'isolation (4) stérile est séparée, du moins dans certaines zones, d'une zone de manœuvre (6) non stérile par un élément de séparation (5) réalisé en particulier sous la forme d'une tablette, dans lequel le dispositif de levage (1) présente au moins une tige de levage (7) comportant une première extrémité (7') qui, à l'état monté, est prévue pour être disposée dans la zone d'isolation (4) stérile, et une seconde extrémité (7") qui, à l'état monté, est prévue pour être disposée à l'extérieur de la zone d'isolation (4), en particulier dans lequel est disposé, dans la zone de la première extrémité (7') de la tige de levage (7), un appareil de manipulation pour la manipulation d'objets dans la zone d'isolation (4), et dans lequel le dispositif de levage (1) est conçu pour déplacer, à l'état monté, la première extrémité (7') de la tige de levage (7) dans la zone d'isolation (4) par un décalage de la tige de levage (7), dans lequel le dispositif de levage (1) comprend un soufflet plissé (8) qui est conçu et disposé de telle sorte que, lors du montage du dispositif de levage (1) sur l'élément de séparation (5), il peut être disposé à l'extérieur de la zone d'isolation (4) stérile, et que le soufflet plissé (8), à l'état monté prévu, entoure une partie de la tige de levage (7), laquelle partie se déplace, lors du décalage de la tige de levage (7), hors de la zone d'isolation (4) dans la zone de manœuvre (6), et la sépare de la zone de manœuvre (6), **caractérisé en ce qu'**une cavité (9) fermée est réalisée entre le soufflet plissé (8) et la tige de levage (7), dans lequel un appareil de rinçage (10) est en outre prévu, lequel comprend une entrée (12) permettant d'introduire un fluide de décontamination, en particulier du peroxyde d'hydrogène, dans la cavité (9) et une sortie (13) permettant d'évacuer le fluide de décontamination hors de la cavité (9).

2. Isolateur (2) comportant une zone d'isolation (4) stérile, dans lequel l'isolateur (2) comprend un élément de séparation (5), dans lequel la zone d'isolation (4) stérile est séparée, du moins dans certaines zones, d'une zone de manœuvre (6) non stérile par l'élément de séparation (5) réalisé en particulier sous la forme d'une tablette, et dans lequel l'isolateur (2) comprend un dispositif de levage (1), **caractérisé en ce que** le dispositif de levage (1) est réalisé selon la revendication 1 et est monté sur l'élément de séparation (5) de telle sorte que le soufflet plissé (8) est disposé à l'extérieur de la zone d'isolation (4) stérile,
**et en ce que** le soufflet plissé (8) entoure une partie de la tige de levage (7), laquelle partie se déplace, lors du décalage de la tige de levage (7), hors de la zone d'isolation (4) dans la zone de manœuvre (6), et la sépare de la zone de manœuvre (6).

3. Installation de production (3) comportant un isolateur (2) selon la revendication 2.

4. Dispositif de levage, isolateur ou installation de production selon l'une des revendications précédentes, **caractérisé en ce que,** à l'état monté prévu, le soufflet plissé (8) est fixé, en particulier de manière amovible, sur un côté extérieur (5') de l'élément de séparation (5) tourné vers la zone de manœuvre (6) non stérile.

5. Dispositif de levage, isolateur ou installation de production selon l'une des revendications précédentes, **caractérisé en ce que** la tige de levage (7) est montée de manière à pouvoir tourner par rapport à l'élément de séparation (5) autour d'un axe de rotation correspondant à l'axe de l'extension longitudinale de la tige de levage (7).

6. Dispositif de levage, isolateur ou installation de production selon la revendication 5, **caractérisé en ce que** le soufflet plissé (8) est monté de manière à pouvoir tourner par rapport à l'élément de séparation (5) et est en particulier disposé de manière à ne pas pouvoir tourner par rapport à la tige de levage (7).

7. Dispositif de levage, isolateur ou installation de production selon la revendication 5, **caractérisé en ce que** le soufflet plissé (8) est monté de manière à pouvoir tourner par rapport à la tige de levage (7) et est en particulier disposé de manière à ne pas pouvoir tourner par rapport à l'élément de séparation (5).

8. Dispositif de levage, isolateur ou installation de production selon l'une des revendications précédentes, **caractérisé en ce que** l'appareil de rinçage (10) présente au moins un clapet antiretour (14) permettant de commander le sens d'écoulement du fluide de décontamination.

9. Dispositif de levage, isolateur ou installation de production selon l'une des revendications précédentes, **caractérisé en ce qu'**un premier clapet antiretour (14') est disposé entre l'entrée (12) et la cavité (9) de telle sorte que, lors d'un décalage de la première extrémité (7') de la tige de levage (7) à l'écart de la zone d'isolation (4), en particulier dans la zone d'isolation (4), le fluide de décontamination s'écoule dans la cavité (9) par l'entrée (12) à travers le premier clapet antiretour (14') et est empêché de refluer par le premier clapet antiretour (14'), **et en ce qu**'un second clapet antiretour (14") est disposé entre la cavité (9) et la sortie (13) de telle sorte que, lors d'un décalage de la première extrémité (7') de la tige de levage (7) vers la zone d'isolation (4), en particulier dans la zone d'isolation (4), le fluide de décontamination s'écoule depuis la cavité (9) hors de la sortie (13) à travers le second clapet antiretour (14") et est empêché de refluer par le second clapet antiretour (14").

10. Dispositif de levage, isolateur ou installation de production selon l'une des revendications précédentes, **caractérisé en ce qu'**est prévu au moins un appareil de transport (15), en particulier une pompe, pour le transport du fluide de décontamination.

11. Dispositif de levage, isolateur ou installation de production selon l'une des revendications précédentes, **caractérisé en ce que** l'entrée (12) et la sortie (13) sont accouplées de manière fluidique à la zone d'isolation (4) stérile.

12. Procédé pour la décontamination d'un dispositif de levage (1), d'un isolateur (2) ou d'une installation de production (3) selon l'une des revendications précédentes, **caractérisé en ce que**
- le fluide de décontamination est transporté dans la cavité (9) à partir d'un réservoir accouplé fluidiquement à l'entrée (12) et à la sortie (13), en particulier de la zone d'isolation (4) stérile, et
- le transport du fluide de décontamination est effectué de manière active par un appareil de transport (15) ou de manière passive par un mouvement de levage de la tige de levage (7) et du soufflet plissé (8).
